# EUROPÄISCHE PATENTANMELDUNG

(11) **EP 1 175 897 A2**
(43) Veröffentlichungstag der Anmeldung: **30.01.2002**
(21) Anmeldenummer: 01116615.4
(22) Anmeldetag: 12.07.2001
(51) Int. Cl.: A61K 7/48

(54) **Kosmetische oder dermatologische Formulierung zur Pflege und Kühlung der Haut nach einem Sonnenbad**

(30) Priorität: 25.07.2000 DE 10036051
(71) Anmelder: Beiersdorf Aktiengesellschaft, 20245 Hamburg (DE)
(72) Erfinder: Kröpke, Rainer, 22869 Schenefeld (DE); Syskowski, Boris, 22303 Hamburg (DE); Grotelüschen, Birgit, 22459 Hamburg (DE)

(57) **Zusammenfassung**

Kosmetische oder dermatologische Formulierungen mit einem Gehalt an
a) mindestens einer flüchtigen Substanz, welche eine Verdunstungszahl von kleiner als 30 hat,
b) mindestens einem Chitosan und
c) mindestens einem Lecithin
und ihre Verwendung.

## Beschreibung

Die vorliegende Erfindung betrifft kühlende kosmetische oder dermatologische Formulierungen, insbesondere kosmetische oder dermatologische Formulierungen, welche die Haut nach einem Sonnenbad gezielt pflegen und die Nachreaktionen der Haut auf die Einwirkung von UV-Strahlung vermindern.

Neben den positiven Auswirkungen des Sonnenlichtes, wie dem allgemeinen Wohlbefinden, der Bildung von Vitamin D3 und der Aknebehandlung, gibt es auch negative Auswirkungen, denen es entgegenzuwirken gilt.

Setzt man die Haut zu lange der Sonne oder einer künstlichen Strahlenquelle aus, so entwickelt sich nach einer Latenzzeit von 2 bis 3 Stunden eine gegen die unbestrahlte Haut stark abgegrenzte Hautrötung, das Erythema solare. Bei dem so entstehenden Sonnenbrand unterscheidet man zwischen
■ 1. Grad: Erythem (Rötung, Wärmegefühl)
   klingt nach 2 bis 3 Tagen wieder ab und verschwindet unter gleichzeitig zunehmender Pigmentierung,
■ 2. Grad: Blasenbildung
   auf der Haut bilden sich Blasen mit Brennen und Jucken, die Oberhaut wird flächig abgestoßen
■ 3. Grad: Zellschädigung
   es treten tiefgehende Zellschädigungen auf, der Körper reagiert mit Fieber, die Oberhaut wird großflächig abgestoßen.

Der 2. und 3. Grad werden auch als Dermatitis solare bezeichnet.

Die Bildung des Erythems ist abhängig von der Wellenlänge. Der Erythembereich des UV-B liegt zwischen 280 nm und 320 nm.

Etwa 90 % der auf die Erde gelangenden ultravioletten Strahlung besteht aus UV-A-Strahlen mit einer Wellenlänge zwischen 320 nm und 400 nm. Während die UV-B-Strahlung in Abhängigkeit von zahlreichen Faktoren stark variiert (z. B. Jahres- und Tageszeit oder Breitengrad), bleibt die UV-A-Strahlung unabhängig von jahres- und tageszeitlichen oder geographischen Faktoren Tag für Tag relativ konstant. Gleichzeitig dringt der überwiegende Teil der UV-A-Strahlung in die lebende Epidermis ein, während etwa 70 % der UV-B-Strahlen von der Homschicht zurückgehalten werden.

Man hat lange Zeit fälschlicherweise angenommen, daß die langwellige UV-A-Strahlung nur eine vemachlässigbare biologische Wirkung aufweist und daß dementsprechend die UV-B-Strahlen für die meisten Lichtschäden an der menschlichen Haut verantwortlich seien. Inzwischen ist allerdings durch zahlreiche Studien belegt, daß UV-A-Strahlung im Hinblick auf die Auslösung photodynamischer, speziell phototoxischer Reaktionen und chronischer Veränderungen der Haut weitaus gefährlicher als UV-B-Strahlung ist. Auch kann der schädigende Einfluß der UV-B-Strahlung durch UV-A-Strahlung noch verstärkt werden.

Da die Beiträge der verschiedenen Wellenlängebereiche des UV-Lichtes zu lichtbedingten Hautveränderungen nicht vollständig geklärt sind, geht man heute verstärkt davon aus, daß vorbeugender Schutz sowohl gegen UV-A- als auch gegen UV-B-Strahlen, beispielsweise durch Auftrag von Lichtschutzfiltersubstanzen in Form einer kosmetischen oder dermatologischen Formulierung auf die Haut, von grundsätzlicher Wichtigkeit ist. Kosmetische oder dermatologische Mittel sollen, in dünner Schicht auf die Haut aufgetragen, diese vor den negativen Auswirkungen der Sonnenstrahlung schützen.

Ein Sonnenbad wird von den meisten Menschen als angenehm empfunden, die nachteiligen Folgen zunächst nicht beachtet. Allerdings hat sich in den letzten Jahren durchaus ein Bewußtsein über die negativen Auswirkungen einer zu intensiven Sonnenbestrahlung herausgebildet, weshalb mehr und stärker schützende Sonnenschutzmittel angewendet werden.

Der Sonnenbrand bzw. das Lichterythem sind die akuten Erscheinungsformen der Lichteinwirkung. Neben den bereits beschriebenen Wirkungen der UV-Strahlen kommt es in der Nachreaktion der Haut femer zu einer verminderten Sebumproduktion und einem Austrocknen der Haut. Zur Linderung und zur Pflege der lichtstrapazierten Haut können deshalb spezielle Wirkstoffe eingesetzt werden, wie beispielsweise
■ Rückfettungs- und Feuchthaltemittel,
■ entzündungslindernde und kühlende Stoffe,
■ lokal anaestesierende Stoffe und/oder
■ desinfizierende Stoffe, um mögliche Hautinfektionen zu verhindern.

Sogenannte Aftersun-Präparate sind dazu bestimmt, die Haut nach dem Sonnenbad zu kühlen und ihr Feuchthaltevermögen zu verbessern, wobei die Vermittlung des Kühleffektes eine zentrale Rolle spielt. Dieser Kühleffekt wird in der Regel durch hohe Mengen an Ethanol erzielt, welches beim Verteilen der Formulierung auf der Haut spontan verdunstet.

Nachteil dieser Formulierungen des Standes der Technik ist allerdings, daß eine langfristige Kühlung nicht erreichbar ist, da das Ethanol sehr schnell verdunstet und die dadurch entstehende Kühlwirkung dementsprechend nur von kurzer Dauer ist.

Der Stand der Technik kennt weitere Zubereitungen, die - auf der Haut oder Schleimhäuten angewandt - befeuchtend und kühlend wirken sollen. In der Literatur werden beispielsweise ionische Verbindungen, insbesondere Ammoniumsalze, als kühlende Agenzien beschrieben. Als kühlende Zubereitungen werden auch verbreitet isopropanolische Gele mit Campher- und Mentholzusatz angewandt sowie überhaupt häufig etherische Öle, vornehmlich Campher und Menthol, aber auch deren Derivate (z. B. Menthyllactat oder Menthyl-3-hydroxybutyrat) in kühlende Zusammensetzungen eingearbeitet werden.

Menthol, Campher und deren Derivate, aber auch andere etherische Öle erniedrigen die Reizschwelle der neuronalen Kälterezeptoren und rufen so ein Kältegefühl hervor. Häufig bewirken sie aber gleichzeitig eine Durchblutungssteigerung, die im Gegenteil ein Wärmegefühl hervorruft. Die Anwendung dieser Substanzen, namentlich auf gereizter Haut, ist jedenfalls problematisch. Darüber hinaus sind viele dieser Verbindungen schlecht wasserlöslich. Ihre Verwendung ist folglich auf wenige Kosmetika und Dermatika beschränkt.

Aufgabe der vorliegenden Erfindung war es also, kosmetische oder dermatologische Zubereitungen zu finden, die die Nachteile des Standes der Technik nicht aufweisen und die insbesondere lichtstrapazierte Haut langanhaltend kühlen.

Es war überraschend und darin liegt die Lösung dieser Aufgaben, daß
kosmetische oder dermatologische Formulierungen mit einem Gehalt an
a) mindestens einer flüchtigen Substanz, welche eine Verdunstungszahl von kleiner als 30 hat,
b) mindestens einem Chitosan und
c) mindestens einem Lecithin
den Nachteilen des Standes der Technik abhelfen würden.

Die erfindungsgemäßen Formulierungen sind in jeglicher Hinsicht überaus befriedigende Präparate, die sich durch eine langanhaltende Kühlwirkung auszeichnen. Es war für den Fachmann nicht vorauszusehen gewesen, daß die erfindungsgemäßen Formulierungen
■ lichtstrapazierte Haut besser pflegen,
■ die Nachreaktionen der Haut auf die Einwirkung von UV-Strahlung besser vermindern,
■ die vom Sonnenbaden gereizte Haut besser beruhigen,
■ leichten Sonnenbrand schneller zum Abklingen bringen würden,
■ besser als feuchtigkeitsspendende Zubereitungen wirken,
■ einfacher zu formulieren sein,
■ besser die Hautglättung fördern,
■ sich durch besser Pflegewirkung auszeichen,
■ besser als Vehikel für kosmetische und medizinisch-dermatologische Wirkstoffe dienen und
■ bessere sensorische Eigenschaften, wie beispielsweise die Verteilbarkeit auf der Haut oder das Einzugsvermögen in die Haut, aufweisen würden als die Zubereitungen des Standes der Technik.

Die Erfindung ist selbstverständlich nicht auf Zubereitungen beschränkt, welche nach dem Sonnenbad angewendet werden, sondern umfaßt naturgemäß alle kosmetischen und dermatologischen Anwendungen, bei welchen eine Kühlwirkung bzw. ein Kühleffekt gewünscht oder von Vorteil sein könnte.

Gegenstand der Erfindung ist daher ferner die
Verwendung von
1. mindestens einem Chitosan und
2. mindestens einem Lecithin
in einer kosmetischen oder dermatologischen Formulierung zur Verzögerung der Verdunstung von flüchtigen Substanzen, welche eine Verdunstungszahl von kleiner als 30 haben, nach dem Auftragen der Formulierung auf die Haut oder die Kopfhaut.

Unter Flüchtigkeit versteht man allgemein das Verdunstungsverhalten bzw. die Verdunstungsgeschwindigkeit von Stoffen, speziell von Flüssigkeiten. Da die Flüchtigkeit realer Flüssigkeiten von vielen Parametern abhängig ist, läßt sie sich kaum theoretisch berechnen. Zur Beurteilung der Flüchtigkeit bedient man sich daher empirisch ermittelter Maßzahlen. Die Verdunstungszahl (VD) ist eine Maßzahl für die Flüchtigkeit. Eine verwandte Kennzahl ist der Verdunstungskoeffizient (VK). Unter der Verdunstungszahl versteht man den Quotienten aus der Verdunstungszeit der zu prüfenden Flüssigkeit und derjenigen von Diethylether als Vergleichsflüssigkeit; als Prüftemperatur gilt 293 ± 2 K, die relative Luftfeuchtigkeit soll bei der Messung 65% ± 5% betragen (vgl. DIN 53170: 1991-08; 53249: 1995-01).

Flüchtige Substanzen im Sinne der vorliegenden Erfindung sind dementsprechend beispielsweise Ethanol (VD = 8,3), Isopropanol (VD = 1,7), n-Hexan (VD = 8,3), n-Heptan (VD = 3,7) und dergleichen mehr.

Besonders vorteilhaft im Sinne der vorliegenden Erfindung sind flüchtige Substanzen, deren Verdunstungszahl zwischen 1 und 30 liegt, insbesondere solche, deren Verdunstungszahl zwischen 1 und 20 liegt.

Chitosan ist gekennzeichnet durch folgende Strukturformel: 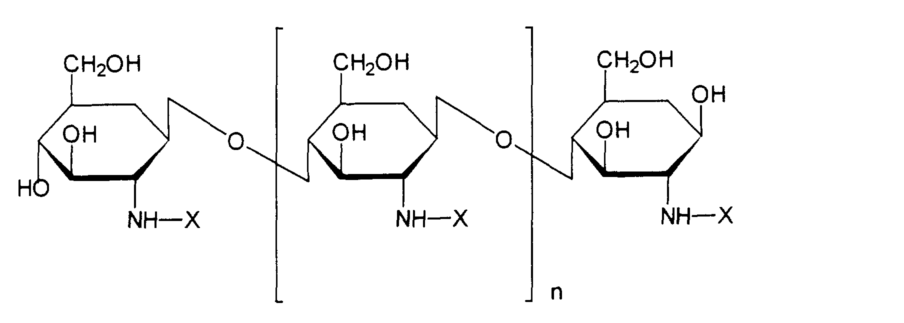 worin n Werte bis zu ca. 2.000 annimmt und X entweder den Acetylrest oder Wasserstoff darstellt. Chitosan entsteht durch Deacetylierung und teilweise Depolymerisation (Hydrolyse) von Chitin, welches durch die Strukturformel 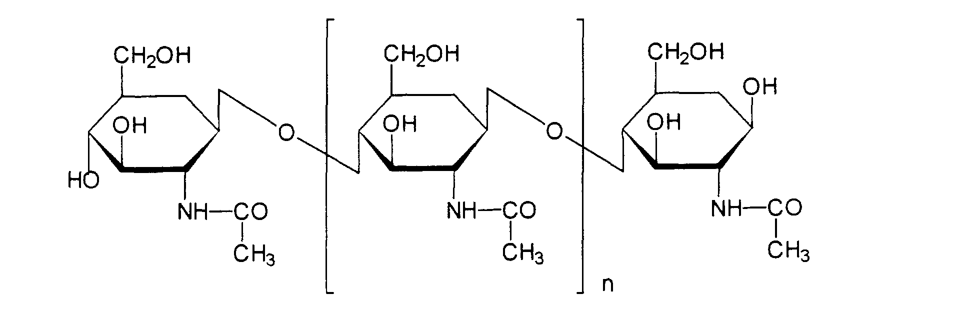 gekennzeichnet ist.

Die Verwendung von Chitosan in Kosmetika ist an sich bekannt. So ist Chitosan z. B. ein in der Haarpflege häufig verwendeter Rohstoff. Es eignet sich, besser als das ihm zugrundeliegende Chitin, als Verdicker oder Stabilisator und verbessert die Adhäsion und Wasserresistenz von polymeren Filmen. Darüber kann Chitosan auch als Moisturizer eingesetzt werden, d. h. man nimmt an, daß Chitosan in der Lage ist, die Feuchtigkeitsabgabe der Hornschicht (auch transepidermal water loss (TEWL) genannt) zu reduzieren und/oder die Hydratation der Hornschicht positiv zu beeinflussen. Stellvertretend für eine Vielzahl von Fundstellen des Standes der Technik: H.P.Fiedler, "Lexikon der Hilfsstoffe für Pharmazie, Kosmetik und angrenzende Gebiete", dritte Auflage 1989, Editio Cantor, Aulendorf, S. 293, Stichwort "Chitosan".

Erfindungsgemäß bevorzugt sind Chitosane mit einem Deacetylierungsgrad von mindestens 50 %, besonders mit einem Deacetylierungsgrad mehr als 60 %, insbesondere mehr als 80%. Unter diesen sind besonders bevorzugt solche, deren 1 %-ige wäßrige Lösung eine maximale Viskosität von 5.000 mPas (Haake Viskotester VT-02 bei 25 °C) aufweist.

Vorteilhafte Chitosane im Sinne der vorliegenden Erfindung sind beispielsweise die unter der Handelsbezeichnung Chitosan L bei der Fa. Primex und die unter den Handelsbezeichnungen Natural Biopolymer Poly+L, Poly+V und Poly+M bei der Fa. Natural Biopolymer erhältlichen.

Vorteilhaft enthalten die erfindungsgemäßen Emulsionen 0,05 bis 5 Gew.-% Chitosan, bevorzugt 0,5 bis 2 Gew.-%, insbesondere 1 Gew.-%, jeweils bezogen auf das Gesamtgewicht der Zubereitungen.

Lecithine gehören zur Gruppe der der Phosphatide (Phospholipoide). Sie zeichen sich durch die allgemeine Struktur 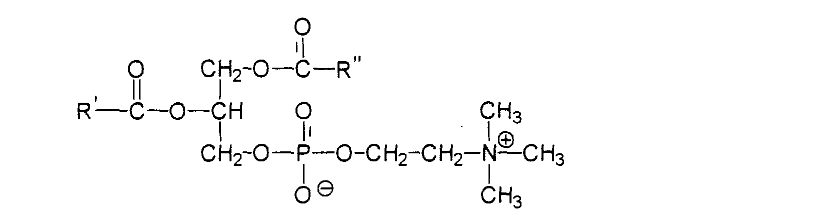 aus, wobei R' und R" typischerweise unverzweigte aliphatische Reste mit 15 oder 17 Kohlenstoffatomen und bis zu 4 cis-Doppelbindungen darstellen.

Aus der Verschiedenheit der Fettsäure-Reste R' und R" ergibt sich eine große Zahl verschiedener Lecithine. Bei der Extraktion aus biologischem Material erhält man immer Gemische. Lecithine werden z. B. aus dem Hühnerei (Ei-Lecithin) oder aus der Sojabohne (Soja-Lecithin) gewonnen. Je nach ihrem Ursprung zeit die Zusammensetzung der Lecithine beträchtliche Unterschiede. So enthält eine Lecithin-Fraktion aus Sojabohnen (dem gebräuchlichsten Rohstoff) z. B. Palmitinsäure, Stearinsäure, Palmitoleinsäure, Oleinsäure, Linolsäure und Linolensäure.

Lecithine sind bräunlichgelbe, etwas hygroskopische, wachsartige Massen, die in Ether und oft auch in Alkohol leicht löslich sind. In Wasser quellen Lecithine wie ein lyophiles Kolloid auf und bilden dann eine durchsichtige, kolloidale Lösung.

Lecithine werden als Emulgatoren hauptsächlich in der Nahrungsmittel-Industrie in Margarine, Schokolade, Backwaren und Überzugsmassen verwendet. Allerdings finden sie auch in kosmetischen Präparaten Verwendung.

Vorteilhafte Lecithine im Sinne der vorliegenden Erfindung sind beispielsweise die unter den Handelsbezeichnungen Sternpur PM, Sternpur E und Nathin 3KE bei der Fa. Stern erhältlichen.

Es ist erfindungsgemäß vorteilhaft, wenn die kosmetischen oder dermatologischen Emulsionen 0,1 bis 5 Gew.-%, insbesondere 0,5 bis 2,5 Gew.-% eines oder mehrerer Lecithine enthalten, jeweils bezogen auf das Gesamtgewicht der Emulsionen.

Die kosmetischen oder dermatologischen Formulierungen im Sinne der vorliegenden Erfindung können bevorzugt neben einer oder mehrerer Ölphasen zusätzlich eine oder mehrere Wasserphasen enthalten und beispielsweise in Form von W/O-, O/W-, W/O/W-oder O/W/O-Emulsionen vorliegen. Solche Emulsionen können vorzugsweise auch eine Mikroemulsion, eine Pickering-Emulsion oder eine sprühbare Emulsion sein.

Vorzugsweise enthalten die erfindungsgemäßen Formulierungen ferner entzündungshemmende Substanzen, wie z. B. Allantoin, α-Bisabolol, Panthotensäure, Panthenol, Gelée Royal, Kamillenextrakte, Azulen oder Aloe-vera-Extrakt sowie unverseifbare Anteile des Avocado- oder Sojaöls und weitere Substanzen, die die gereizte Haut beruhigen. Weitere vorteilhafte Wirkstoffe sind Gerbstoffe, welche adstringierende, entzündungshemmende und/oder sekretionshemmende Wirkung haben.

Darüberhinaus können die erfindungsgemäßen Formulierungen auch vorteilhaft Dihydroxyaceton oder Nußextrakte enthalten sowie weitere Substanzen, welche die Bräune erhalten sollen.

Die erfindungsgemäßen kosmetischen und/oder dermatologischen Formulierungen können wie üblich zusammengesetzt sein und insbesondere zur Behandlung und der Pflege der Haut und/oder der Haare nach einem Sonnenbad und als Schminkprodukt in der dekorativen Kosmetik dienen. Entsprechend können die erfindungsgemäßen Formulierungen - je nach ihrem Aufbau - beispielsweise verwendet werden als Hautschutzcreme, Reinigungsmilch, Sonnenschutzlotion, Nährcrème, Tages- oder Nachtcrème usw. Es ist gegebenenfalls möglich und vorteilhaft, die erfindungsgemäßen Formulierungen als Grundlage für pharmazeutische Formulierungen zu verwenden. Bevorzugt sind insbesondere solche kosmetischen und dermatologischen Formulierungen, die in der Form eines sogenannten Aftersun-Hautpflegeproduktes vorliegen.

Zur Anwendung werden die erfindungsgemäßen kosmetischen und dermatologischen Formulierungen in der für Kosmetika üblichen Weise auf die Haut und/oder die Haare in ausreichender Menge aufgebracht.

Die erfindungsgemäßen kosmetischen und dermatologischen Formulierungen können kosmetische Hilfsstoffe enthalten, wie sie üblicherweise in solchen Zubereitungen verwendet werden, z. B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel und/oder Silikonderivate sowie Moisturizer.

Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Homschicht positiv zu beeinflussen.

Vorteilhafte Moisturizer im Sinne der vorliegenden Erfindung sind beispielsweise Glycerin, Milchsäure, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist.

Die jeweils einzusetzenden Mengen an kosmetischen oder dermatologischen Hilfs- und Trägerstoffen und Parfüm können in Abhängigkeit von der Art des jeweiligen Produktes vom Fachmann durch einfaches Ausprobieren leicht ermittelt werden.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z. B. Glycin, Histidin, Tyrosin, Tryptophan) und deren Derivate, Imidazole (z. B. Urocaninsäure) und deren Derivate, Peptide wie D,L-Carnosin, D-Carnosin, L-Camosin und deren Derivate (z. B. Anserin), Carotinoide, Carotine (z. B. α-Carotin, β-Carotin, Lycopin) und deren Derivate, Chlorogensäure und deren Derivate, Liponsäure und deren Derivate (z. B. Dihydroliponsäure), Aurothioglucose, Propylthiouracil und andere Thiole (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z. B. pmol bis µmol/kg), ferner (Metall)-Chelatoren (z. B. α-Hydroxyfettsäuren, Palmitinsäure, Phytinsäure, Lactoferrin), α-Hydroxysäuren (z. B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z. B. γ-Linolensäure, Linolsäure, Ölsäure), Folsäure und deren Derivate, Ubichinon und Ubichinol und deren Derivate, Vitamin C und Derivate (z. B. Ascorbylpalmitat, Mg-Ascorbylphosphat, Ascorbylacetat), Tocopherole und Derivate (z. B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Hamsäure und deren Derivate, Mannose und deren Derivate, Zink und dessen Derivate (z. B. ZnO, ZnSO₄) Selen und dessen Derivate (z. B. Selenmethionin), Stilbene und deren Derivate (z. B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Emulsionen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Die Lipidphase kann vorteilhaft gewählt werden aus folgender Substanzgruppe:
- Mineralöle, Mineralwachse
- Öle, wie Triglyceride der Caprin- oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C-Zahl, z. B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wäßrige Phase der erfindungsgemäßen Formulierungen enthält gegebenenfalls vorteilhaft
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte, ferner Alkohole niedriger C-Zahl, z. B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Xanthangummi und/oder Hydroxypropylmethylcellulose, jeweils einzeln oder in Kombination.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiele

| **Beispiel 1:** | Gew.-% |
|---|---|
| Chitosan | 1,0 |
| Lecithin | 1,0 |
| Glycerin | 3,0 |
| Milchsäure (90 %ige wässrige Lösung)¹ | 0,6 |
| Parfüm, Konservierungsmittel, Farbstoffe, Antioxidantien etc. | q.s. |
| Ethanol | 35,0 |
| Wasser | ad 100% |
| pH-Wert | ca. 4,5 |

| **Beispiel 2:** | |
|---|---|
| | Gew.-% |
| Chitosan | 0,1 |
| Lecithin | 3,0 |
| Glycerin | 15,0 |
| Milchsäure (90%ige wässrige Lösung)¹ | 0,06 |
| Ethanol | 70,0 |
| Parfüm, Konservierungsmittel, Farbstoffe, Antioxidantien etc., Natronlauge | q.s. |
| Wasser | ad 100% |
| | |
| pH-Wert | ca. 5,5 |

| | |
|---|---|
| ¹ Es können auch alle anderen Säuren verwendet werden, die lösliche Chitosansalze ergeben, wie z. B. Phosphorsäue, Essigsäure, Ascorbinsäure (unter Schutzgas), Salzsäure, Glykolsäure, Salpetersäure, 2-Pyrolidon-5-carbonsäure, Apfelsäure, Salicylsäure, Benzoesäure, Malonsäure, Bernsteinsäure, Adipinsäure, Glutarsäure und/oder Glutaminsäure. Weiterhin kann durch Veränderung des Chitosan-/Säure-Verhältnisses der pH-Wert im Bereich von 3,5 bis 5,5 variiert werden. | |

| **Beispiel 3:** | |
|---|---|
| | Gew.-% |
| Chitosan | 3,0 |
| Lecithin | 1,5 |
| Propylenglycol | 5,0 |
| Milchsäure (90 %ige wässrige Lösung)¹ | 0,6 |
| | |
| Ethanol | 17,5 |
| Isopropanol | 7,5 |
| | |
| Parfüm, Konservierungsmittel, Farbstoffe, Antioxidantien etc. | q.s. |
| Wasser | ad 100% |
| | |
| pH-Wert | ca. 4,5 |

| **Beispiel 4:** | |
|---|---|
| | Gew.-% |
| Chitosan | 1,0 |
| Lecithin | 2,0 |
| Glycerin | 20,0 |
| Milchsäure (90 %ige wässrige Lösung)¹ | 0,6 |
| Parfüm, Konservierungsmittel, Farbstoffe, Antioxidantien etc. | q.s. |
| Cyclomethicon | 50,0 |
| Polysorbate® 20 (Uniqema)² | 2,0 |
| Wasser | ad 100% |
| pH-Wert | ca. 4,5 |

| | |
|---|---|
| ² Lösungsvermittler mit einem HLB-Wert größer als 15 | |

| **Beispiel 5:** | |
|---|---|
| | Gew.-% |
| Chitosan | 1,0 |
| Lecithin | 1,0 |
| Glycerin | 20,0 |
| Milchsäure (90 %ige wässrige Lösung)¹ | 0,6 |
| Parfüm, Konservierungsmittel, Farbstoffe, Antioxidantien etc. | q.s. |
| iso-Pentan | 10,0 |
| Wasser | ad 100% |
| | |
| pH-Wert | ca. 4,5 |

| **Beispiel 6 :** | |
|---|---|
| | Gew.-% |
| Chitosan | 1,5 |
| Lecithin | 2,5 |
| Glycerin | 20,0 |
| Milchsäure (90 %ige wässrige Lösung)¹ | 0,6 |
| Parfüm, Konservierungsmittel, Farbstoffe, Antioxidantien etc. | q.s. |
| Ethanol | 30,0 |
| Dimethicone | 5,0 |
| Cyclomethicone | 15,0 |
| | |
| pH-Wert | ca. 4,5 |

## Patentansprüche

1. Kosmetische oder dermatologische Formulierungen mit einem Gehalt an
a) mindestens einer flüchtigen Substanz, welche eine Verdunstungszahl von kleiner als 30 hat,
b) mindestens einem Chitosan und
c) mindestens einem Lecithin.

2. Kosmetische oder dermatologische Formulierungen nach Anspruch 1, **dadurch gekennzeichnet, daß** sie zusätzlich Wirkstoffe gewählt aus der Gruppe entzündungshemmende Substanzen enthalten.

3. Kosmetische oder dermatologische Formulierungen nach einem der Ansprüche 1 oder 2, **dadurch gekennzeichnet, daß** sie zusätzlich Rückfettungs- und Feuchthaltemittel enthalten.

4. Kosmetische oder dermatologische Formulierungen nach einem der Ansprüche 1 bis 3, **dadurch gekennzeichnet, daß** sie in Form einer Mehrfachemulsion, Mikroemulsion, Pickering-Emulsion oder sprühbarer Emulsion vorliegen.

5. Kosmetische oder dermatologische Formulierungen nach einem der vorhergehenden Ansprüche **dadurch gekennzeichnet, daß** die Verdunstungszahl der flüchtigen Substanz(en) zwischen 1 und 20 liegt.

6. Verwendung einer kosmetischen oder dermatologischen Formulierung nach einem der Ansprüch 1 bis 4 zur Pflege lichtstrapazierter Haut und/oder zur Linderung oder der Nachreaktionen der Haut auf die Einwirkung von UV-Strahlung.

7. Verwendung von
a) mindestens einem Chitosan und
b) mindestens einem Lecithin in einer kosmetischen oder dermatologischen Formulierung zur Verzögerung der Verdunstung von flüchtigen Substanzen, welche eine Verdunstungszahl von kleiner als 30 haben, nach dem Auftragen der Formulierung auf die Haut oder die Kopfhaut.

8. Kosmetisches oder dermatologisches Sonnenschutzset, mindestens enthaltend jeweils eine kosmetische oder dermatologische Formulierung A und B, **dadurch gekennzeichnet, daß**
a) Formulierung A mindestens eine Filtersubstanz enthält, welche UV-A- und/oder UV-B-Strahlen filtert,
b) Formulierung B
i) mindestens eine flüchtige Substanz, welche eine Verdunstungszahl von kleiner als 30 hat,
ii) mindestens ein Chitosan und
iii) mindestens ein Lecithin enthält, wobei
c) die Formulierungen A und B physikalisch voneinander getrennt vorliegen und separat voneinander aus einem Mehrkammerpackmittel entnehmbar sind.
